# EUROPEAN PATENT SPECIFICATION

(11) **EP 0 935 709 B1**
(45) Date of publication and mention of the grant of the patent: **25.02.2004**
(21) Application number: 98941107.9
(22) Date of filing: 31.08.1998
(51) Int. Cl.: F04B 43/08, F04B 43/00

(54) **IMPROVED ACCURACY PERISTALTIC PUMP**
PERISTALTISCHE PUMPE MIT VERBESSERTER GENAUIGKEIT
POMPE PERISTALTIQUE DE PRECISION AMELIOREE

(30) Priority: 04.09.1997 GB 9718686
(43) Date of publication of application: 18.08.1999
(73) Proprietor: BAXTER INTERNATIONAL INC., Deerfield, IL 60015 (US)
(72) Inventor: DANBY, Hal C., Suffolk CO10 0PZ (GB)
(74) Representative: Dee, Ian Mark
(86) International application number: PCT/US1998/018055
(87) International publication number: WO 1999/011932

(56) References cited:
- EP-A- 0 388 596
- US-A- 4 585 442
- US-A- 4 671 792
- US-A- 4 893 991

## Description

### BACKGROUND OF THE INVENTION

### Technical Field

The present invention is directed toward liquid delivery devices for controlling the flow of liquid from a liquid reservoir, and more particularly toward an improved accuracy peristaltic pump.

### Background Art

Peristaltic pumps are particularly suited for use in accurately metering and infusing fluids such as medications into the bodies of hospital patients. Heminway, U.S. Patent No. 4,893,991, discloses a linear type of peristaltic pump. Natwick, U.S. Patent No. 5,055,001, discloses a valve/plunger type of peristaltic pump. Galea, U.S. Patent No. 3,999,891, discloses a rotary type peristaltic pump. Each of these peristaltic pumps operate to propel liquid through a resilient tubing which is typically made from a flexible plastic material such as polyvinyl chloride or the like. The tubing is repeatedly compressed and expanded along a defined section of the tubing. The tube is typically expanded or "rebounded" by its internal resiliency. A known problem with this type of pump is that the portions of tubing which are intermittently compressed and expanded tend to fatigue with time. As a result, the tubing is less able to rebound to its original cross-section when released, decreasing the volume of the tubing along the critical pump segment and thereby degrading pump accuracy. U.S. Patent No. 4,893,991 notes that such pumps have been found to exhibit as much as a 10% drop in flow rate in a 24 hour period.

The prior art has recognized this short coming in peristaltic pumps and has attempted at least three ways to solve the problem. First, Heminway, U.S. Patent No. 4,893,991, attempts to improve pump accuracy by preventing the portion of resilient tubing which is subject to compression and expansion from assuming a cylindrical configuration upon expansion. That is, the plungers which compress and expand the tubing are designed to maintain the segment of tubing in an oval cross-section even at full expansion. A principal problem with the solution set forth in Heminway is that it requires very accurate tolerances with the plungers in the retracted position so that the tubing expands to a consistent oval cross-section in order for the pump to operate at accurate rates and volumes. In addition, because the tubing is not able to assume its full circular cross-section, and therefore its greatest volume, Heminway unduly restricts the rate liquid can be pumped.

Natwick, U.S. Patent No. 5,055,001, proposes an even more complicated solution to improving accuracy in peristaltic pumps. Natwick proposes that the range of diametric compression of the tubing be from about 15% with the plunger retracted to about 85% with the plunger extended. Natwick argues that since the tubing need never recover to a fully uncompressed condition, changes in the elasticity of the flexible tubing due to continued use and repeated compression have much less effect on the volumetric capacity of the pump. In addition, because the plunger never fully compresses the pumping portion of the tubing, the tubing is subjected to less fatigue. Natwick further teaches providing mechanical tubing shapers disposed on each side of the plunger which are extended to reform the pumping portion of the tubing as the plunger is retracted and the tubing refills with fluid. Natwick suffers from the same shortfall of Heminway in that it restricts the volume of the tubing used for pumping and therefore limits pump output rates. In addition, the tubing shapers are complex mechanical structures which create an additional avenue for potential pump failure. Moreover, the mechanical shapers taught in Natwick require a number of potentially costly parts and complicate the assembly of the pump.

Mannes, U.S. Patent No. 4,585,442, discloses an intravenous infusion rate controller which operates on a resilient tubing which rests in a trough between a pair of resilient bands. The resilient bands act on opposing sides of the outer diameter of the tubing in a compressed state to aid in restoring the tube to its original cross-section upon expansion. In this manner the resilient bands inhibit the tendency of the tube to "flatten out" and rebound to only an oval cross-section which degrades the accuracy of the rate controller. Unfortunately, the resilient bands act on only two discrete points in attempting to restore the tubing to its original shape. Moreover, the space between the resilient bands must be maintained at rather precise tolerances to avoid the resilient bands compressing the tubing into an oval cross-section if the bands are too close or failing to restore the tube to its circular cross-section if the bands are too far apart.

The present invention is directed toward overcoming one or more of the problems discussed above.

According to the present invention, there is provided a liquid delivery device according to claim 1 and an elastomeric sleeve according to claim 10 for use in a liquid delivery device.

### SUMMARY OF THE INVENTION

A first aspect of the invention is a liquid delivery device which controls the flow of liquid from a liquid reservoir. The liquid delivery device includes a resilient tubing having a cylindrical wall with a substantially circular cross-section defining a flow lumen. The flow lumen is in fluid communication with the reservoir. A compression member selectively compresses a lengthwise segment of the cylindrical wall to collapse the flow lumen and releases the lengthwise segment to open the flow lumen. An elastomeric sleeve envelopes greater than half an outer periphery of the cross-section of the resilient tubing along at least a portion of the lengthwise segment of the cylindrical wall. The elastomeric sleeve biases the lengthwise segment of the resilient tube to restore it to its substantially circular cross-section when the compression member releases the lengthwise segment.

The elastomeric sleeve, in a relaxed state, preferably has an inner diameter less than an outer diameter of the resilient tubing. The elastomeric sleeve preferably has a lengthwise substantially linear opening of a width less than half the cross-section of the sleeve so that a portion of the resilient tubing can be received lengthwise within the elastomeric sleeve with more than half the periphery of the tubing cross-section enveloped by the sleeve. As the lengthwise segment of the resilient tube is collapsed, the opening of the elastomeric sleeve can expand to release the lengthwise segment of the tube. The liquid delivery device preferably further includes an integral handle along the length of the elastomeric sleeve and means such as a a clamp for grasping the handle to fix the position of the elastomeric sleeve relative to the compression member.

A second aspect of the invention is a liquid delivery device for delivering a flow of liquid from a reservoir through elongate resilient tubing. The resilient tubing has a cylindrical wall with a substantially circular cross-section defining a flow lumen. The flow lumen is in fluid communication with the reservoir. The liquid delivery device comprises an elongate elastomeric sleeve which, in a relaxed state, has an inner diameter less than an outer diameter of the resilient tubing. The elastomeric sleeve has a lengthwise substantially linear opening of a width less than half of the cross-section of the sleeve so that a portion of the resilient tubing can be received lengthwise within the elastomeric sleeve with more than half the periphery of the tubing cross-section enveloped by the sleeve. A plurality of spaced reciprocating plungers are arranged substantially linearly along the elastomeric sleeve. The reciprocating plungers extend and retract relative to the elastomeric sleeve. An anvil is disposed opposite each of the plurality of plungers, adjacent the opening of the elastomeric sleeve and spaced from the plungers so that, with the resilient tubing received in the elastomeric sleeve, the reciprocating plungers collapse the flow lumen against the anvil with the plungers extended and do not collapse the flow lumen with the plungers retracted. As the plungers are retracted so as to no longer collapse the flow lumen, the elastomeric sleeve biases the resilient tubing received therein to its substantially circular cross-section. Preferably, as the segment of the resilient tubing is collapsed, the opening of the elastomeric sleeve expands to release the resilient tubing and the resilient tubing is in direct contact with the anvil. The elastomeric sleeve preferably includes an integral handle along its length and the peristaltic pump preferably includes a means such as a clamp for grasping the handle to fix the position of the elastomeric sleeve relative to the plungers. The resilient tube may be held in position relative to the plungers and anvil substantially solely by the elastomeric sleeve.

In a preferred embodiment, the elastomeric sleeve, in cross-section, is an extended semi-circle with a lengthwise handle extending along each end of the extended semi-circle and the peristaltic pump includes means, e.g. clamps, for grasping the handles to fix the position of the elastomeric sleeve relative to the plungers.

A third aspect of the present invention is an elastomeric sleeve for use in a liquid delivery device with a resilient tubing having a cylindrical wall with a substantially circular cross-section. The liquid delivery device controls the flow of liquid from a liquid reservoir through the resilient tubing by a compression member selectively compressing and releasing a lengthwise segment of the tubing to collapse and restore a lumen of the tubing. The elastomeric sleeve comprises means for housing the resilient tubing and an elongate side wall which is arcuate in cross-section and which in a relaxed state has an inner diameter less than an outer diameter of the resilient tubing. The side wall is dimensioned so that it envelopes greater than half an outer periphery of the cross-section of the resilient tubing along at least a portion of a lengthwise segment of the cylindrical wall. The sleeve further comprises a lengthwise substantially linear opening having a width to allow a portion of the resilient tubing to be received lengthwise within the elastomeric sleeve. A lengthwise handle extends from the side wall for securing the sleeve in position in the liquid delivery device. The elastomeric sleeve is adapted to restore the lengthwise segment of the resilient tubing to its substantially circular cross-section following compression thereof by a compression member of the liquid delivery device. Preferably, the side wall and the handle are integrally formed of a single piece of elastomer.

The elastomeric sleeve of the present invention subjects the majority of the wall of resilient tubing to a uniform compression which serves to bias and restore the tubing to its substantially circular cross-section. In this manner, the segment of tubing subject to compression and expansion pumps liquid at a constant rate over extended periods of time. In addition, the sleeve helps secure the tubing in a desired position relative to the compression member or plunger and anvil to ease loading of the tubing into the pumping device and to help secure the tubing during pumping. The elongate opening in the sleeve expands during compression of the tubing segment so that the tubing directly connects the anvil, which causes compression of the tubing between the rigid anvil and the plunger. This decreases the amount of energy necessary to collapse the lumen (as opposed to having to compress the entire elastomeric sleeve between the plunger and anvil), thereby improving pumping efficiency. Furthermore, the sleeve is readily manufactured out of inexpensive materials and is easy to instal.

### Brief Description of the Drawings

Fig. 1 is a perspective view of a peristaltic pump housing including an elastomeric sleeve in accordance with the present invention;
Fig. 2 is a cross-sectional view taken along line 2-2 of Fig. 1 with the pump housing door shut and a plunger in a retracted position;
Fig. 3 is the same as Fig. 2 only the plunger is shown in an extended position;
Fig. 4 is a cross-sectional view taken along line 4-4 of Fig. 1 showing the elastomeric sleeve and resilient tubing operatively associated with the linear peristaltic pump apparatus;
Fig. 5 is an alternate embodiment of Fig. 4 showing a valve/plunger of a peristaltic pump replacing the linear peristaltic pump of Fig. 4 and with an alternate embodiment of the elastomeric sleeve;
Fig. 6 is a cross-sectional view taken along line 6-6 of Fig. 5; and
Fig. 7 is a cross-sectional view taken along line 7-7 of Fig. 5.

### Detailed Description of the Preferred Embodiment

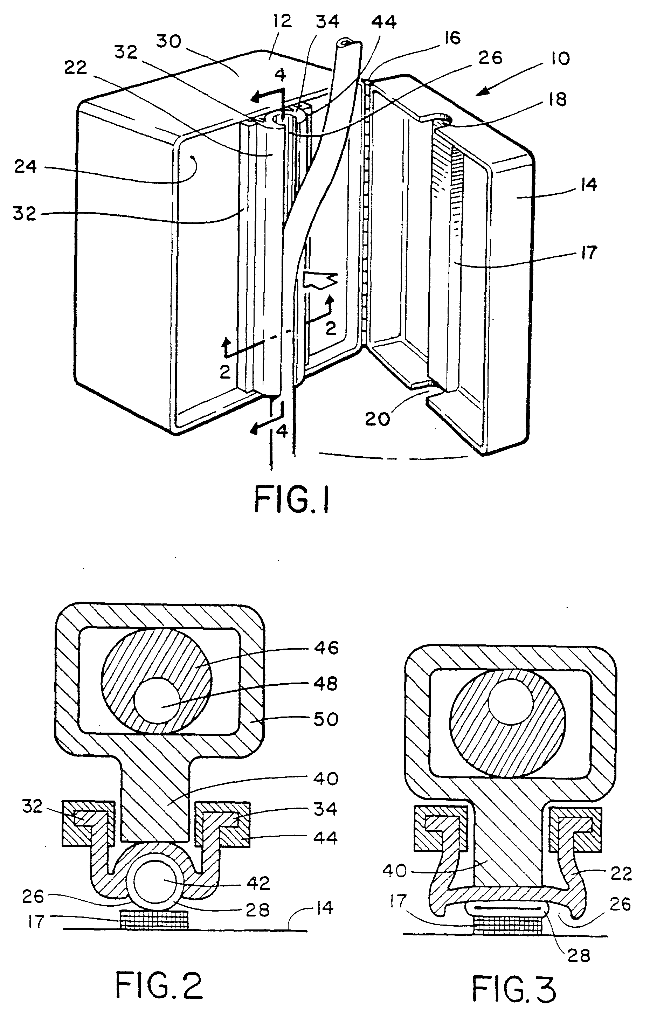

An improved accuracy peristaltic pump 10 is shown in perspective view in Fig. 1. Most of the features not important to the understanding of this invention such as controls, latch mechanisms and the like have been omitted for the sake of clarity. The improved accuracy peristaltic pump 10 includes a housing base 12 and a housing door 14 joined by a hinge 16. An anvil 17 is attached to an inner surface of the housing door 14 and runs lengthwise of the door 14. The door 14 further includes a pair of annular slots 18, 20 in its top and bottom side walls. While the specific embodiment disclosed herein is of a peristaltic type pump, the elastomeric sleeve can also provide its many advantages in other flow control devices such as the intravenous infusion rate controller disclosed in Mannes. U.S. Patent No. 4,585,442.

An elastomeric sleeve 22 is attached to a partition 24 within the housing base 12 and runs the length of the housing base 12. The elastomeric sleeve 22 is positioned relative to the anvil 17 so that with the door 14 closed, the surface of the anvil 17 aligns with an elongate opening 26 of the elastomeric sleeve 22. As seen in Figs. 1 and 2. the elastomeric sleeve 22 consists of a substantially cylindrical side wall 30 with the lengthwise opening 26 removed from the side wall. A pair of handles 32, 34 extend lengthwise along the elastomeric sleeve 22 and project tangentially from adjacent the lengthwise slot 26. Referring to Fig. 2, the elastomeric sleeve in cross-section is an extended semi-circle with each handle 32, 34 extending tangentially along each end of the semi-circle. The elastomeric sleeve 22, including the handles 32, 34 is integrally formed of a single piece of a suitable resilient. deformable elastomer, such as rubber, preferably a room temperature vulcanizing (RTV) silicone.

The elastomeric sleeve 22 is dimensioned so that the inner diameter of the cylindrical side wall is slightly smaller than the outer diameter of the resilient tubing 28. In addition, the width of the elongate slot 26 is less than one half the cross-section of the elastomeric sleeve 22. In this manner, as the resilient tubing 28 is installed as illustrated in Fig. 1, the resilient tubing 28 is held in place. As best shown in Fig. 2, the elastomeric sleeve 22 biases the resilient tubing 28 to restore it to a substantially circular cross-sectional shape wherein the flow lumen 42 is unrestricted and the plunger 40 is restricted. Referring to Fig. 2 and as shown with regard to the handle 34 in Fig. 1. the handles 32, 34 are secured by elongate clamps 44.

As seen in Fig. 2, the plunger 40 is actuated by a cam 46 mounted eccentrically to a drive shaft 48. the cam residing in a collar 50 attached to the plunger 40. In Fig. 2 the plunger is in a retracted position. The plunger 40 is illustrated in its fully extended position in Fig. 3. Here the plunger 40 is shown having fully collapsed the lumen 42 of the resilient tubing 28. In addition, the plunger 40 causes the elongate slot 26 to expand and release the tubing 28. In this manner, the tubing 28 is compressed directly against the anvil 17 as it is disengaged from the elastomeric sleeve 22.

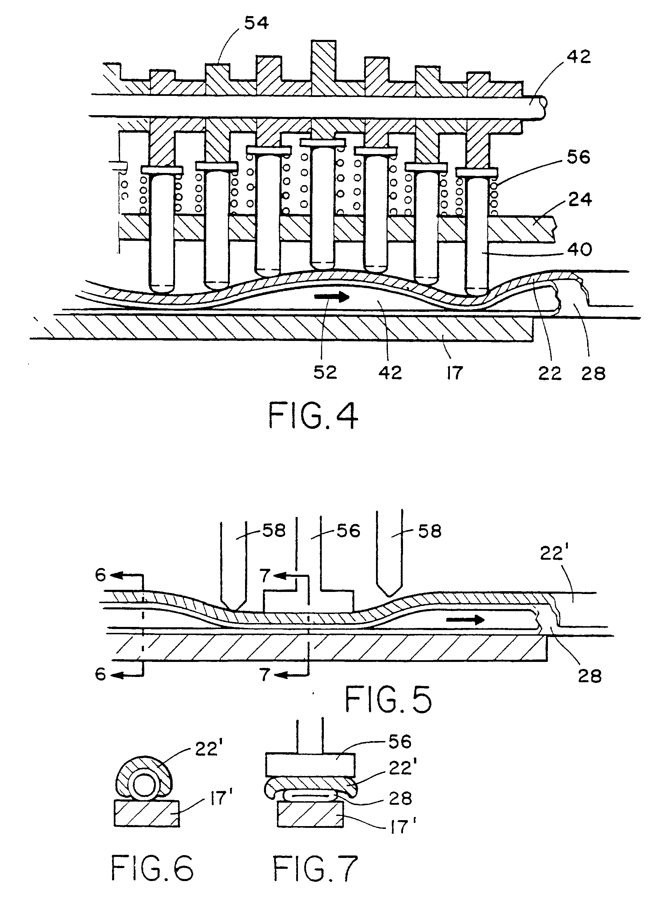

In Fig. 4 the door 14 is closed and the resilient tubing 28 and the sleeve 22 are in an operative position relative to the anvil 17 and plungers 40. The plungers 40 are sequentially extended and retracted relative to the tubing 28 and elastomeric sleeve 22 to propel fluid through the lumen 42 as indicated by the arrow 52. The operation of a linear peristaltic pump as illustrated Fig. 4 is described in detail in Heminway, U.S. Patent No. 4,893,991. The plunger mechanism illustrated in Fig. 4 differs from that illustrated in Figs. 2 and 3 in that instead of having eccentric cams 54 mounted within a collar 50 to direct the plunger between its extended and retracted position, springs 56 bias the plungers toward a retracted position and the eccentric cams 54 extend the plungers 40 as they rotate around the drive shaft 42.

Fig. 5 illustrates another embodiment of the elastomeric sleeve 22 used in conjunction with a valve/plunger type peristaltic pump. The elastomeric sleeve 22' does not include the elongate handles 32, 34, but is in all other manners identical to the elastomeric sleeve 22 described above with regard to Figs. 1-4. In this embodiment the resilient tubing 28 resides within the elastomeric sleeve 22' and is held in place by a length of the elastomeric sleeve 22' which is not subjected to compression, as illustrated in Fig. 6. When subject to compression, just as described above with regard to Fig. 3, the elastomeric sleeve 22' disengages the resilient tubing 28 so it may be compressed directly against the anvil 17'. Because of the absence of the lengthwise handles 32, 34, the sleeve 22' must be secured to the housing in some other manner. For example, the ends of the sleeve may be fastened to the housing. Also, although not illustrated in Fig. 5, the elastomeric sleeve 22' need not extend continuously between the plunger 56 and the valves 58. The elastomeric sleeve need only extend along a portion of the tubing subject to compression a distance sufficient to restore the portion of tubing back to the substantially cylindrical cross-section.

The improved accuracy peristaltic pump features an elastomeric sleeve which envelopes greater than half of the outer periphery of a resilient tubing to bias the tubing to a substantially cylindrical cross-section in a relaxed state. By providing substantially uniform compression about the periphery of the resilient tubing, the resilient tubing is reliably repeatedly restored to the substantially cylindrical shape. Thus, the elastomeric sleeve insures the continued accuracy of a liquid delivery device such as a peristaltic.pump that controls fluid flow by repeated compression and release of a resilient tubing. The elastomeric sleeve also secures the resilient tubing which both assists in retaining the resilient tubing in an operative position relative to the plunger and anvil and also provides a convenient way for insuring the tube is properly loaded in the pump housing prior to a fluid delivery operation. Because the sleeve releases the tubing during extension of the plunger, the tubing is compressed directly against the anvil 17, minimizing energy consumption by the pump. Moreover, these many advantages are provided by an elastomeric sleeve that is inexpensively fabricated out of a variety of elastomers and installed in a pump for minimal cost.

## Claims

1. A liquid delivery device (10) for controlling the flow of liquid from a liquid reservoir, the liquid delivery device comprising:
a resilient tubing (28) having a cylindrical wall with a substantially circular cross-section defining a flow lumen (42), the flow lumen being in fluid communication with the reservoir;
a compression member for selectively compressing a lengthwise segment of the cylindrical wall to collapse the flow lumen (42) and releasing the lengthwise segment to open the flow lumen; and
an elastomeric sleeve (22) enveloping greater than half an outer periphery of the cross-section of the resilient tubing (28) along at least a portion of the lengthwise segment of the cylindrical wall, the elastomeric sleeve biasing the lengthwise segment of the resilient tubing to its substantially circular cross-section when the compression member releases the lengthwise segment.

2. The liquid delivery device of claim 1, wherein the elastomeric sleeve (22), in a relaxed state, has an inner diameter less than an outer diameter of the resilient tubing (28), the elastomeric sleeve further having a lengthwise substantially linear opening (26) of a width so that a portion of the resilient tubing can be received lengthwise within the elastomeric sleeve with more than half the periphery of the tubing cross-section enveloped by the sleeve.

3. The liquid delivery device of claim 2, wherein the lengthwise opening (26) is of a width sufficient that as the lengthwise segment of the resilient tubing (28) is collapsed, the lengthwise opening expands to release the lengthwise segment of the resilient tubing.

4. The liquid delivery device of any one of claims 1 to 3 further comprising an integral handle (32, 34) along the length of the elastomeric sleeve (22) and means for grasping the handle to fix the position of the elastomeric sleeve relative to the compression member.

5. The liquid delivery device of claim 1, wherein the resilient tubing (28) is elongate; the elastomeric sleeve (22) is elongate and, in a relaxed state, has an inner diameter less than an outer diameter of the elongate resilient tubing, the elastomeric sleeve having a lengthwise substantially linear opening (26) of a width so that a portion of the resilient tubing can be received lengthwise within the elastomeric sleeve with more than half the periphery of the tubing cross-section enveloped by the sleeve; and
the compression member comprises a plurality of spaced reciprocating plungers (40) arranged substantially linearly along the elastomeric sleeve, the reciprocating plungers extending and retracting relative to the elastomeric sleeve; and an anvil (17) disposed opposite each of the plurality of plungers, adjacent the linear opening of the elastomeric sleeve and spaced from the plungers so that, with the resilient tubing received in the elastomeric sleeve, the reciprocating plungers collapse the flow lumen against the anvil with the plungers extended and do not collapse the flow lumen with the plungers retracted,
whereby as the plungers are retracted so as to no longer collapse the flow lumen, the elastomeric sleeve biases the resilient tubing received therein to its substantially circular cross-section.

6. The liquid delivery device of claim 5, wherein the lengthwise opening (26) is of a width sufficient that as the lengthwise segment of the resilient tubing (28) is collapsed, the lengthwise opening expands to release the resilient tubing and the resilient tubing is in direct contact with the anvil (17).

7. The liquid delivery device of claim 5 or claim 6 further comprising an integral handle (32, 34) along the length of the elastomeric sleeve (22) and means for grasping the handle to fix the position of the elastomeric sleeve relative to the plungers (40).

8. The liquid delivery device of claim 7, wherein the resilient tubing (28) is held in position relative to the plungers (40) and anvil (17) substantially solely by the elastomeric sleeve (22).

9. The liquid delivery device of any one of claims 5 to 8, wherein the elastomeric sleeve (22), in cross-section, is an extended semi-circle with a lengthwise handle (32, 34) extending tangentially along each end of the extended semi-circle, the liquid delivery device further comprising means for grasping the handles to fix the position of the elastomeric sleeve relative to the plungers,(40).

10. An elastomeric sleeve (22) for use in a liquid delivery device according to claim 1, said elastomeric sleeve having:
means for housing a resilient tubing (28) having a cylindrical wall with a substantially circular cross-section;
an elongate side wall (30) which is arcuate in cross-section and which, in a relaxed state, has an inner diameter less than an outer diameter of the resilient tubing and is dimensioned so that it envelopes greater than half an outer periphery of the cross-section of the resilient tubing along at least a portion of a lengthwise segment of the cylindrical wall;
a lengthwise substantially linear opening (26) having a width to allow a portion of the resilient tubing to be received lengthwise within the elastomeric sleeve;
a lengthwise handle (32, 34) extending from the side wall for securing the sleeve in position in the liquid delivery device,
wherein the elastomeric sleeve is adapted to restore the lengthwise segment of the resilient tubing to its substantially circular cross-section following compression thereof by a compression member of the liquid delivery device.

11. The elastomeric sleeve of claim 10, wherein the side wall (30) and the handle (32, 34) are integrally formed of a single piece of elastomer.

12. The elastomeric sleeve of claim 10, further comprising a pair of lengthwise handles (32, 34) each extending tangentially from the side wall (30) along a side of the lengthwise opening (26).

13. The elastomeric sleeve of claim 10 or claim 11, wherein the side wall (30) is an extended semi circle in cross-section.

14. The elastomeric sleeve of claim 13, further comprising a pair of lengthwise handles (32, 34) each extending tangentially along each end of the extended semi-circle.

## Patentansprüche

1. Flüssigkeitsabgabevorrichtung (10) zum Steuern des Flüssigkeitsdurchflusses aus einem Flüssigkeitsreservoir, wobei die Flüssigkeitsabgabevorrichtung folgendes aufweist:
- einen elastischen Schlauch (28), der eine zylindrische Wand mit einem im wesentlichen kreisförmigen Querschnitt hat, der ein Durchflußlumen (42) bildet, wobei das Durchflußlumen mit dem Reservoir in Fluidverbindung ist;
- ein Druckelement, um ein Längssegment der zylindrischen Wand selektiv zusammenzudrücken, damit das Durchflußlumen (42) kollabiert, und um das Längssegment freizugeben, damit das Durchflußlumen geöffnet wird; und
- eine elastomere Hülse (22), die mehr als die Hälfte eines Außenumfangs des Querschnitts des elastischen Schlauchs (28) zumindest entlang einem Bereich des Längssegments der zylindrischen Wand umhüllt, wobei die elastomere Hülse das Längssegment des elastischen Schlauchs in seinen im wesentlichen kreisförmigen Querschnitt vorspannt, wenn das Druckelement das Längssegment freigibt.

2. Flüssigkeitsabgabevorrichtung nach Anspruch 1,
wobei die elastomere Hülse (22) in einem entspannten Zustand einen Innendurchmesser hat, der kleiner als ein Außendurchmesser des elastischen Schlauchs (28) ist, wobei die elastomere Hülse ferner eine in Längsrichtung im wesentlichen lineare Öffnung (26) mit einer solchen Breite hat, daß ein Bereich des elastischen Schlauchs in Längsrichtung innerhalb der elastomeren Hülse aufgenommen werden kann, wobei mehr als die Hälfte des Umfangs des Schlauchquerschnitts von der Hülse umhüllt wird.

3. Flüssigkeitsabgabevorrichtung nach Anspruch 2,
wobei die Längsöffnung (26) eine Breite hat, die ausreicht, damit dann, wenn das Längssegment des elastischen Schlauchs (28) kollabiert, sich die Längsöffnung aufweitet, um das Längssegment des elastischen Schlauchs freizugeben.

4. Flüssigkeitsabgabevorrichtung nach einem der Ansprüche 1 bis 3,
die ferner eine integrale Handhabe (32, 34) entlang der Länge der elastomeren Hülse (22) und eine Einrichtung zum Greifen der Handhabe aufweist, um die Position der elastomeren Hülse relativ zu dem Druckelement zu fixieren.

5. Flüssigkeitsabgabevorrichtung nach Anspruch 1,
wobei der elastische Schlauch (28) langgestreckt ist; wobei die elastomere Hülse (22) langgestreckt ist und in einem entspannten Zustand einen Innendurchmesser hat, der kleiner als ein Außendurchmesser des langgestreckten elastischen Schlauchs ist, wobei die elastomere Hülse eine in Längsrichtung im wesentlichen lineare Öffnung (26) mit einer solchen Breite hat, daß ein Bereich des elastischen Schlauchs in Längsrichtung innerhalb der elastomeren Hülse aufgenommen werden kann,
wobei mehr als die Hälfte des Umfangs des Schlauchquerschnitts von der Hülse umhüllt wird; und
wobei das Druckelement folgendes aufweist: eine Vielzahl von voneinander beabstandeten hin- und hergehenden Kolben (40), die im wesentlichen linear entlang der elastomeren Hülse angeordnet sind, wobei die hin- und hergehenden Kolben relativ zu der elastomeren Hülse ausfahren und einfahren; und einen Amboß (17), der jedem von der Vielzahl von Kolben gegenüberliegend, der linearen Öffnung der elastomeren Hülse benachbart und von den Kolben beabstandet angeordnet ist, so daß dann, wenn der elastische Schlauch in der elastomeren Hülse aufgenommen ist, die hin- und hergehenden Kolben das Durchflußlumen gegen den Amboß zum Kollabieren bringen, wenn die Kolben ausgefahren sind, und das Durchflußlumen nicht zum Kollabieren bringen, wenn die Kolben eingefahren sind,
so daß dann, wenn die Kolben eingefahren sind, so daß sie das Durchflußlumen nicht mehr zum Kollabieren bringen, die elastomere Hülse den darin aufgenommenen elastischen Schlauch in seinen im wesentlichen kreisförmigen Querschnitt vorspannt.

6. Flüssigkeitsabgabevorrichtung nach Anspruch 5,
wobei die Längsöffnung (26) eine Breite hat, die ausreicht, damit dann, wenn das Längssegment des elastischen Schlauchs (28) kollabiert, sich die Längsöffnung aufweitet, um den elastischen Schlauch freizugeben, und der elastische Schlauch in direktem Kontakt mit dem Amboß (17) ist.

7. Flüssigkeitsabgabevorrichtung nach Anspruch 5 oder 6,
die ferner eine integrale Handhabe (32, 34) entlang der Länge der elastomeren Hülse (22) und eine Einrichtung zum Greifen der Handhabe aufweist, um die Position der elastomeren Hülse relativ zu den Kolben (40) zu fixieren.

8. Flüssigkeitsabgabevorrichtung nach Anspruch 7,
wobei der elastische Schlauch (28) relativ zu den Kolben (40) und dem Amboß (17) im wesentlichen nur von der elastomeren Hülse (22) in Position gehalten wird.

9. Flüssigkeitsabgabevorrichtung nach einem der Ansprüche 5 bis 8,
wobei die elastomere Hülse (22) im Querschnitt ein erweiterter Halbkreis ist, wobei sich eine in Längsrichtung verlaufende Handhabe (32, 34) tangential entlang jedem Ende des erweiterten Halbkreises erstreckt, wobei die Flüssigkeitsabgabevorrichtung ferner eine Einrichtung zum Greifen der Handhaben aufweist, um die Position der elastomeren Hülse relativ zu den Kolben (40) zu fixieren.

10. Elastomere Hülse (22) zum Gebrauch in einer Flüssigkeitsabgabevorrichtung nach Anspruch 1,
wobei die elastomere Hülse folgendes aufweist:
- eine Einrichtung zur Aufnahme eines elastischen Schlauchs (28), der eine zylindrische Wand mit einem im wesentlichen kreisförmigen Querschnitt hat;
- eine langgestreckte Seitenwand (30), die im Querschnitt bogenförmig ist und in einem entspannten Zustand einen Innendurchmesser hat, der kleiner als ein Außendurchmesser des elastischen Schlauchs ist, und die so dimensioniert ist, daß sie mehr als die Hälfte eines Außenumfangs des Querschnitts des elastischen Schlauchs zumindest entlang einem Bereich eines Längssegments der zylindrischen Wand umhüllt;
- eine in Längsrichtung im wesentlichen lineare Öffnung (26) mit einer solchen Breite, daß ein Bereich des elastischen Schlauchs in Längsrichtung innerhalb der elastomeren Hülse aufgenommen werden kann;
- eine in Längsrichtung verlaufende Handhabe (32, 34), die sich von der Seitenwand aus erstreckt, um die Hülse in der Flüssigkeitsabgabevorrichtung in ihrer Position festzulegen;
- wobei die elastomere Hülse so ausgebildet ist, daß sie das Längssegment des elastischen Schlauchs im Anschluß an dessen Zusammendrücken durch ein Druckelement der Flüssigkeitsabgabevorrichtung in seinen im wesentlichen kreisförmigen Querschnitt zurückbringt.

11. Elastomere Hülse nach Anspruch 10,
wobei die Seitenwand (30) und die Handhabe (32, 34) aus einem einzigen Elastomerstück integral gebildet sind.

12. Elastomere Hülse nach Anspruch 10,
die ferner ein Paar von in Längsrichtung verlaufenden Handhaben (32, 34) aufweist, die sich tangential von der Seitenwand (30) entlang einer Seite der Längsöffnung (26) erstrecken.

13. Elastomere Hülse nach Anspruch 10 oder 11,
wobei die Seitenwand (30) im Querschnitt ein erweiterter Halbkreis ist.

14. Elastomere Hülse nach Anspruch 13,
die ferner ein Paar von in Längsrichtung verlaufenden Handhaben (32, 34) aufweist, die sich tangential entlang jedem Ende des erweiterten Halbkreises erstrecken.

## Revendications

1. Dispositif de distribution de liquide (10) pour commander l'écoulement de liquide à partir d'un réservoir de liquide, le dispositif de distribution de liquide comprenant:
un tube élastique (28) ayant une paroi cylindrique de section transversale sensiblement circulaire définissant un passage intérieur d'écoulement (42), le passage d'écoulement étant en communication de fluide avec le réservoir,
un élément de compression pour comprimer sélectivement un segment longitudinal de la paroi cylindrique afin d'écraser le passage d'écoulement (42), et pour libérer le segment longitudinal afin d'ouvrir le passage d'écoulement ; et
un manchon en élastomère (22) qui enveloppe plus de la moitié d'une périphérie extérieure de la section transversale du tube élastique (28) le long d'au moins une partie du segment longitudinal de la paroi cylindrique, le manchon en élastomère rappelant le segment longitudinal du tube élastique à sa section transversale sensiblement circulaire lorsque l'élément de compression libère le segment longitudinal.

2. Dispositif de distribution de liquide selon la revendication 1, dans lequel le manchon en élastomère (22), dans un état relâché, a un diamètre intérieur plus petit qu'un diamètre extérieur du tube élastique (28), le manchon en élastomère comportant en outre une ouverture longitudinale sensiblement linéaire (26) d'une largeur telle qu'une partie du tube élastique peut être reçue longitudinalement dans le manchon en élastomère tandis que plus de la moitié de la périphérie de la section transversale du tube est enveloppée par le manchon.

3. Dispositif de distribution de liquide selon la revendication 2, dans lequel l'ouverture longitudinale (26) est d'une largeur suffisante pour que, lorsque le segment longitudinal du tube élastique (28) est comprimé, l'ouverture longitudinale s'élargisse de manière à libérer le segment longitudinal du tube élastique.

4. Dispositif de distribution de liquide selon une quelconque des revendications 1 à 3, comprenant en outre une poignée solidaire (32, 34) le long du manchon en élastomère (22), et des mo yens de prise de la poignée pour fixer la position du manchon en élastomère par rapport à l'élément de compression.

5. Dispositif de distribution de liquide selon la revendication 1, dans lequel le tube élastique (28) est allongé, le manchon en élastomère (22) est allongé et il a, dans un état relâché, un diamètre intérieur plus petit qu'un diamètre extérieur du tube élastique allongé, le manchon en élastomère comportant une ouverture longitudinale sensiblement linéaire (26) d'une largeur telle qu'une partie du tube élastique peut être reçue longitudinalement dans le manchon en élastomère tandis que plus de la moitié de la périphérie de la section transversale du tube est enveloppée par le manchon ; et
l'élément de compression comprend une pluralité de plongeurs à mouvement alternatif espacés (40) agencés sensiblement linéairement le long du manchon en élastomère, les plongeurs à mouvement alternatif étant mis en extension et en rétraction par rapport au manchon en élastomère ; et une enclume (17) disposée en face de chacun de la pluralité de plongeurs, de façon adjacente à l'ouverture linéaire du manchon en élastomère et espacée des plongeurs de sorte que, le tube élastique étant logé dans le manchon en élastomère, les plongeurs à mouvement alternatif compriment le passage d'écoulement contre l'enclume lorsque les plongeurs sont en extension, et ne compriment pas le passage d'écoulement lorsque les plongeurs sont rétractés,
de sorte que, lorsque les plongeurs sont rétractés de manière à ne plus comprimer le passage d'écoulement, le manchon en élastomère rappelle le tube élastique logé dans le manchon à sa section transversale sensiblement circulaire.

6. Dispositif de distribution de liquide selon la revendication 5, dans lequel l'ouverture longitudinale (26) est d'une largeur suffisante pour que, lorsque le segment longitudinal du tube élastique (28) est comprimé, l'ouverture longitudinale s'élargisse de manière à libérer le tube élastique, le tube élastique étant alors en contact direct avec l'enclume (17).

7. Dispositif de distribution de liquide selon la revendication 5 ou la revendication 6, comprenant en outre une poignée solidaire (32, 34) le long du manchon en élastomère (22), et des moyens de prise de la poignée afin de fixer la position du manchon en élastomère par rapport aux plongeurs (40).

8. Dispositif de distribution de liquide selon la revendication 7, dans lequel le tube élastique (28) est maintenu en position par rapport aux plongeurs (40) et à l'enclume (17) sensiblement seulement par le manchon en élastomère (22).

9. Dispositif de distribution de liquide selon une quelconque des revendications 5 à 8, dans lequel le manchon en élastomère (22), en section transversale, est un demi-cercle prolongé comportant une poignée longitudinale (32, 34) qui s'étend tangentiellement le long de chaque extrémité du demi-cercle prolongé, le dispositif de distribution de liquide comprenant en outre des moyens de prise des poignées pour fixer la position du manchon en élastomère par rapport aux plongeurs (40).

10. Manchon en élastomère (22) utilisable dans un dispositif de distribution de liquide selon la revendication 1, le dit manchon en élastomère ayant:
un moyen de logement d'un tube élastique (28) à paroi cylindrique de section transversale sensiblement circulaire ;
une paroi latérale allongée (30) qui est de section transversale courbe et qui a, dans un état relâché, un diamètre intérieur plus petit qu'un diamètre extérieur du tube élastique et qui est dimensionnée de sorte qu'elle enveloppe plus de la moitié d'une périphérie extérieure de la section transverale du tube élastique le long d'au moins une partie d'un segment longitudinal de la paroi cylindrique;
une ouverture longitudinale sensiblement linéaire (26) ayant une largeur qui permet de recevoir une partie du tube élastique longitudinalement dans le manchon en élastomère ;
une poignée longitudinale (32, 34) s'étendant à partir de la paroi latérale pour fixer le manchon en position dans le dispositif de distribution de liquide ;
dans lequel le manchon en élastomère est prévu pour ramener le segment longitudinal du tube élastique à sa section transversale sensiblement circulaire après sa compression par un élément de compression du dispositif de distribution de liquide.

11. Manchon en élastomère selon la revendication 10, dans lequel la paroi latérale (30) et la poignée (32, 34) sont formées solidairement en une seule pièce en élastomère.

12. Manchon en élastomère selon la revendication 10, comprenant en outre deux poignées longitudinales (32, 34) s'étendant chacune tangentiellement à partir de la paroi latérale (30) le long d'un côté de l'ouverture longitudinale (26).

13. Manchon en élastomère selon la revendication 10 ou la revendication 11, dans lequel la paroi latérale (30) est un demi-cercle prolongé en section transversale.

14. Manchon en élastomère selon la revendication 13, comprenant en outre deux poignées longitudinales (32, 34) s'étendant chacune tangentiellement le long de chaque extrémité du demi-cercle prolongé.
